(19) Europäisches Patentamt
European Patent Office
Office européen des brevets

(11) **EP 3 682 921 B1**

(12) **EUROPÄISCHE PATENTSCHRIFT**

(45) Veröffentlichungstag und Bekanntmachung des Hinweises auf die Patenterteilung:
**01.03.2023 Patentblatt 2023/09**

(21) Anmeldenummer: **20151354.6**

(22) Anmeldetag: **13.01.2020**

(51) Internationale Patentklassifikation (IPC):
**A61M 5/31** (2006.01)   **A61M 5/28** (2006.01)

(52) Gemeinsame Patentklassifikation (CPC):
**A61M 5/3129;** A61M 5/284; A61M 2005/3132

(54) **VORFÜLLBARE SPRITZE**

PREFILLABLE SYRINGE

SERINGUE PRÉREMPLISSABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.01.2019 DE 102019101279**

(43) Veröffentlichungstag der Anmeldung:
**22.07.2020 Patentblatt 2020/30**

(73) Patentinhaber: **Gerresheimer Bünde GmbH 32257 Bünde (DE)**

(72) Erfinder: **Zeiß, Bernd 49324 Melle (DE)**

(74) Vertreter: **Hannke, Christian Hannke Bittner & Partner Patent- und Rechtsanwälte mbB Prüfeninger Straße 1 93049 Regensburg (DE)**

(56) Entgegenhaltungen:
**WO-A1-2017/055462     DE-A1- 10 110 126 JP-A- 2004 129 695**

## Beschreibung

Die Erfindung betrifft eine vorfüllbare Spritze für medizinische Anwendungen mit einen hohlzylindrischen sich entlang einer axialen Achse (X) erstreckenden Spritzenkörper, an dessen distalem Ende ein hohlzylindrisches Endstück angeformt ist, welches einen Durchtrittskanal aufweist, dessen Durchmesser kleiner ist als ein Innendurchmesser des Spritzenkörpers, wobei der Spritzenkörper einen Vorratsbereich für ein zur Verabreichung vorgesehenes Medium umfasst. Patentdokumente WO 2017/055462 A1, JP 2004 129695 A und DE 101 10 126 A1 offenbaren Spritzen mit zwei in zwei Kammern befindlichen Medien.

Vorgefüllte Spritzen werden üblicherweise mit dem zur Anwendung kommenden Medium an den Nutzer ausgeliefert. Diese Art von Spritzen hat verschiedene Vorteile. Zum einen ist die Handhabung der Spritzen sehr einfach, da das Medium nicht vor der Anwendung in die Spritze transferiert werden muss. Weiterhin ist, selbst im Notfall, die Wahrscheinlichkeit der Anwendung eines falschen Medikaments sehr gering. Derartige Spritzen kommen weltweit in der Pharma- und Biotech-Industrie zur Anwendung. Für Impfstoffe und zahlreiche andere Medikamente sind sie heutzutage das Primärpackmittel erster Wahl. Die vorfüllbaren Spritzen(-körper) werden von dem Primärpackmittelhersteller unter Reinraumbedingungen hergestellt, abgepackt, sterilisiert und abfüllfertig direkt in den Reinraum des Abfüllers geliefert. Sie können dann ohne weitere Behandlungsschritte zur Abfüllung eingesetzt werden. Die vorfüllbaren Spritzen werden durch den Abfüller von der proximalen Seite, bzw. der Flanschseite befüllt.

Die genannten vorfüllbaren Spritzen können aus Glas oder aus Kunststoff gefertigt werden. Die Spritzen können auch bereits mit einem Stechmittel ausgestattet sein. Ein solches Stechmittel kann beispielsweise mittels einer Klebeverbindung in dem Durchtrittskanal befestigt werden.

Die Lagerung der Spritzen kann bei bestimmten zur Verabreichung vorgesehenen Medien problematisch sein, da das Medium mit Verunreinigungen oder anderweitigen Stoffen in Kontakt treten. Insbesondere bei modernen Arzneimitteln und biotechnologischen Wirkstoffen kann ein solcher, während der Lagerung lang andauernder Kontakt zu unerwünschten Reaktionen führen, welche das Präparat unbrauchbar machen. Ein solcher unerwünschter Kontakt kann beispielsweise ein Kontakt mit dem Klebemittel, mittels welchem die Nadel in die Durchtrittsöffnung eingeklebt ist, sein. Ferner kann für manche in der Spritze gelagerten Medien der Kontakt mit der metallischen Nadel nachteilhaft sein.

Bei der Herstellung von Glasspritzen erfolgt die Ausformung des Endstücks durch abschnittsweises Aufheizen des Glasrohlings. Ist der Glasrohling in einem formbaren Zustand, erfolgt die Formgebung mittels eines in den Glasrohling eingeführten Formstifts.

Während die Formwerkzeuge auf der Außenseite der Röhre unkritisch sind, da ein eventuell entstehender Abrieb nicht mit dem Füllgut in Kontakt kommt, sind alle Werkzeuge, welche innerhalb des Glasrohlings Anwendung finden, kritisch zu betrachten. Der Formstift besteht in der Regel aus Wolfram. Der Formstift für den Kanal des Spritzenkonus ist sehr klein und heizt sich durch die geringe Wärmekapazität stark auf, so dass Oxide entstehen, die zu Verschleiß und Abrieb führen. Der Abrieb des Formstifts liegt in einer Größenordnung, die es erforderlich macht, dass der Formstift in der Regel nach ca. 1 Stunde Betrieb ausgetauscht werden muss. Dieser Abrieb schlägt sich zumindest anteilig in dem Kanal nieder. Dort kann er später durch das Füllgut absorbiert werden und eventuell zur Beeinträchtigung von dessen Wirksamkeit und/oder Verträglichkeit führen. Gelöste Wolfram-Polyanione in vorgefüllten Spritzen interagieren mit einigen Proteinen, so dass diese aggregieren. Partikelbildung und eine Änderung der Wirksamkeit des Arzneimittels oder körpereigener Proteine sind die Folge. Insbesondere neuartige biotechnologische Wirkstoffe weisen oft langkettige Molekülketten auf, welche sehr empfindlich hinsichtlich einer Wechselwirkung mit Spurenelementen ist. Diese Produkte können somit nicht in normalen Spritzen gelagert werden.

Durch ein Extraktionsverfahren und nachfolgender analytischer Bestimmung sind die Rückstände durch den Abrieb nachweisbar. Ausgehend von Unverträglichkeitsvorfällen in der Industrie hat bereits eine Verständigung auf zulässige Grenzwerte stattgefunden. Demnach werden nach derzeitigem Stand Spritzen mit einem Wolfram-Gehalt von < 50 ng als hochreine Spritzen bezeichnet. Standard-Spritzen können dagegen Werte zwischen 500 und 2500 ng aufweisen.

In der Industrie werden derzeit Lösungen durch Waschprozesse oder Ersatz-Metalle wie Platin bereitgestellt. Diese können den Gehalt aber nur reduzieren, bzw. bringen ein Ersatzmetall ein, welches seinerseits auch nicht frei von Wechselwirkungen sein muss.

Aufgabe der vorliegenden Erfindung ist es demnach eine vorfüllbare Spritze bereitzustellen, mittels welcher die oben genannten Nachteile überwunden werden können.

Die Aufgabe wird gelöst von einer vorfüllbaren Spritze für medizinische Anwendungen mit einen hohlzylindrischen, sich entlang einer axialen Achse (X) erstreckenden Spritzenkörper, an dessen distalem Ende ein hohlzylindrisches Endstück ausgeformt ist, welches einen Durchtrittskanal aufweist, wobei der Spritzenkörper einen Vorratsbereich für ein zur Verabreichung vorgesehenes Medium umfasst, wobei in einem Ruhezustand der Vorratsbereich durch einen distalen Beabstandungsbereich von dem Durchtrittskanal des hohlzylindrischen Endstücks entlang der axialen Achse (X) beabstandet ist und ein erster Stopfen den Vorratsbereich von dem distalen Beabstandungsbereich trennt, wobei in einem Abgabezustand durch eine Verlagerung des ersten Stopfens in den distalen Beabstandungsbereich eine fluidische Verbindung für das Medium zwischen dem Vorratsbereich und dem Durchtrittskanal über einen Bypasskanal öffenbar ist, wobei sich eine axiale Länge des Bypasskanals zumindest über eine axiale Länge des distalen Beabstandungsbereichs erstreckt.

Durch die Beabstandung des zur Verabreichung vorgesehenen Mediums von dem hohlzylindrischen Endstück wird während der Lagerung der Kontakt mit drei potenziell riskanten Kontaktmaterialien verhindert. Zum einen wird bei einer vorzugsweise vorliegenden Glasspritze der Kontakt zu eventuellen Wolframrückständen in dem Durchtrittskanal verhindert. Bei vorzugsweise vorgesehenen Spritzen mit einem eingeklebten Stechmittel (sogenannte "staked in needle" (SIN)) wird der Kontakt zu dem Metall (Edelstahl) des Stechmittels und/oder der Kontakt zu dem Klebemittel unterbunden. In dem Abgabezustand kann das Medium durch den Durchtrittskanal des Endstücks treten. Das Medium tritt somit nur kurz während der Injektion in Kontakt mit o.g. Materialien. Dies ist jedoch unbedenklich. Ein längerdauernder Kontakt und damit auch mögliche Interaktionen werden jedoch durch die erfindungsgemäße Spritze vermieden.

Unter einer fluidischen Verbindung ist ein Transfer der des flüssigen Mediums von dem Vorratsbereich durch den Bypasskanal zu verstehen.

Die Begriffe "distal" und "proximal" sind derart zu verstehen, dass das distale Ende der Spritze näher an dem Applikationsort ist und das proximale Ende weiter weg von dem Applikationsort. Analog sind die Begriffe "distale und proximale Richtung" zu verstehen.

Unter einem Stechmittel sind Nadeln, Kanülen und ähnliche Mittel für derartige Zwecke zu verstehen.

Erfindungsgemäß ist an dem distalen Ende des Spritzenkörpers ein hohlzylindrisches Endstück ausgeformt. Vorteilhafterweise weist der Spritzenkörper einen Hauptabschnitt auf. Bevorzugt ist der Hauptabschnitt als ein Hohlkörper mit kreisförmiger Grundfläche ausgebildet. Weiterhin ist es vorteilhaft, wenn ein Übergangsbereich zwischen dem Hauptabschnitt und dem Endstück vorgesehen ist. Vorteilhafterweise ist der distale Beabstandungsbereich ein Teil des Hauptabschnitts und schließt an dem Übergangsbereich an.

Vorteilhafterweise ist ein Außendurchmesser des Hauptabschnitts größer als ein Außendurchmesser des Endstücks, wobei bevorzugt der Außendurchmesser des Hauptabschnitts über seine gesamte axiale Länge, ausgenommen im Bereich des Bypasses konstant ist. Weiterhin ist es vorteilhaft, wenn ein Innendurchmesser des Hauptabschnitts größer ist als ein Durchmesser des Durchtrittskanals. Auch hier ist es bevorzugt, wenn der Innendurchmesser des Hauptabschnitts über seine gesamte axiale Länge ausgenommen im Bereich des Bypasses konstant ist. Der Außendurchmesser und der Innendurchmesser des Endstücks können nach einer vorteilhaften Ausgestaltung ebenfalls über die axiale Länge des Endstücks konstant sein. Es sind jedoch auch Ausführungsformen vorgesehen, in welchen der Außendurchmesser und der Innendurchmesser des Endstücks nicht über die axiale Ausdehnung des Endstücks konstant sind. In dem Übergangsbereich findet demnach ein vorteilhafter Übergang von dem größeren Außendurchmesser des Hauptabschnitts auf den kleineren Außendurchmesser des Endstücks statt. Ebenfalls findet in dem Übergangsbereich ein vorteilhafter Übergang von dem größeren Innendurchmesser des Hauptabschnitts zu dem kleineren Durchmesser des Durchtrittskanals statt.

Nach einer weiteren vorteilhaften Ausführungsform ist an dem proximalen Ende des Hauptabschnitts eine Fingerauflage vorgesehen. Ferner ist an dem proximalen Ende des Spritzenkörpers eine proximale Öffnung des Spritzenkörpers vorgesehen. An dem distalen Ende des Endstücks ist weiterhin eine distale Öffnung vorgesehen, in welche der Durchtrittskanal mündet.

Nach einer weiteren vorteilhaften Ausführungsform ist an einer Innenwand des Hauptabschnitts eine Gleitschicht angebracht. Durch eine solche Gleitschicht ist ein geringerer Kraftaufwand notwendig, um den zweiten und oder den ersten Stopfen in dem Spritzenkörper zu verlagern. Gleichzeitig wird aber auch ein ausreichend dichtes Anliegen des ersten und/oder zweiten Stopfens gewährleistet. Vorzugsweise ist eine solche Gleitschicht eine Silikonenthaltenden Beschichtung. Denkbar wäre jedoch auch eine silikonfreie Beschichtung.

Nach einer besonders bevorzugten Ausführungsform ist der Bypasskanal eine in radialer Richtung (R) nach außen gerichtete Auswölbung des Spritzenkörpers. Vorteilhafterweise grenzt ein distales Ende des Bypasskanals im Wesentlichen an dem Übergangsbereich an. Unter im Wesentlichen ist hier zu verstehen, dass ein möglichst geringer Abstand zwischen dem Übergangsbereich und dem distalen Ende des Bypasskanals vorliegen soll. Der distale Beabstandungsbereich und somit auch der Bypasskanal sollten möglichst nahe an dem Endstück angeordnet sein, damit das Volumen beziehungsweise die Aufnahmekapazität des Vorratsbereichs maximiert werden können. Aus herstellungstechnischen Gründen ist ein geringer, im Grunde vernachlässigbarer Toleranzabstand, zu dem Übergangsbereich beziehungsweise der Wölbung in diesem Bereich vorhanden.

Vorteilhafterweise ist der Bypasskanal in der vorderen distalen Hälfte des Hauptabschnitts angeordnet. Bevorzugt ist der Bypasskanal in dem vorderen distalen Drittel des Hauptabschnitts angeordnet. Bevorzugt ist der Bypasskanal in dem vorderen distalen Viertel des Hauptabschnitts angeordnet. Bevorzugt ist der Bypasskanal in dem vorderen distalen Achtel des Hauptabschnitts angeordnet.

Vorzugsweise besitzt der Hauptabschnitt ein Volumen von 0,5 ml oder 1ml oder 1-3ml oder 5 ml oder größer. Durch das Vorsehen eines distalen Beabstandungsbereichs ist das eigentliche Fassungsvermögen für das Medium kleiner, wobei durch die vorteilhafte Anordnung des Bypasses nahe des Übergangsbereichs die Länge des distalen Beabstandungsbereichs minierbar ist und somit das Fassungsvermögen des Vorratsbereichs maximiert werden kann.

Nach einer bevorzugten Ausführungsform entspricht die axiale Länge des distalen Beabstandungsbereichs einer axialen Länge des ersten Stopfens und einer Länge eines ersten und eines zweiten Übergriffsbereichs. Im Abgabezustand befindet sich der erste Stopfen in dem distalen Beabstandungsbereich. Vorzugsweise ist dieser im Wesentlichen mittig in dem distalen Beabstandungsbereich beziehungsweise mittig zu dem Bypasskanal angeordnet. Ein Übergriffsbereich ist somit ein Bereich des Bypasses, welcher im Abgabezustand über den sich im distalen Beabstandungsbereich befindlichen ersten Stopfens hinausragt. Demnach kann am proximalen Ende des Bypasskanals, beziehungsweise durch den zweiten Übergangsbereich das Medium in den Bypasskanal eintreten und am distalen Ende des Bypasskanals, beziehungsweise durch den zweiten Übergangsbereich das Medium aus dem Bypasskanal austreten.

Nach einer bevorzugten Ausführungsform ist der Vorratsbereich durch einen zweiten Stopfen in proximaler Richtung (X2) begrenzt. Es ist weiterhin bevorzugt, dass zumindest ein Mantelbereich des zweiten Stopfens aus einem elastischen Material besteht. Der zweite Stopfen könnte somit vollständig aus einem elastischen Material sein oder einen nichtelastischen Kern aufweisen, welcher von einem Mantelbereich aus einem elastischen Material umgeben ist. Vorteilhafterweise wird beim Einbringen des zweiten Stopfens in den Hohlraum des Spritzenkörpers das elastische Material komprimiert, so dass der zweite Stopfen dichtend an einer Innenwand des hohlzylindrischen Spritzenkörpers anliegt. Weiterhin ist es bevorzugt, dass der zweite Stopfen an einem distalen Ende eine distale Endfläche aufweist, welche planar ausgebildet ist. Durch eine solche planare Ausgestaltung der distalen Endfläche kann gewährleistet werden, dass das Medium, welches sich zwischen dem ersten und zweiten Stopfen befindet, vollständig aus dem zwischenliegenden Bereich verdrängt werden kann. Weiterhin ist es vorteilhaft, wenn der zweite Stopfen an einem proximalen Ende eine Sackbohrung aufweist, in welchem eine Kolbenstange anordenbar ist. Vorzugsweise ist die Sackbohrung mit einem Innengewinde versehen, mittels welchem die Kolbenstange befestigbar ist. Vorzugsweise ist das Innengewinde ein ISO 10040-5 Gewinde.

Nach einer bevorzugten Ausführungsform ist der zweite Stopfen in dem hohlzylindrischen Spritzenkörper entlang der axialen Achse (X) verlagerbar. Vorteilhafterweise ist durch eine Verlagerung des zweiten Stopfens in distaler Richtung (X1) die Verlagerung des ersten Stopfens in den distalen Beabstandungsbereich hervorrufbar. In dem Abgabezustand wird demnach ein Kraft, vorzugsweise durch eine Kolbenstange, auf den zweiten Stopfen ausgeübt, so dass dieser sich in distaler Richtung (X1) verlagert. In der Regel kann das, sich in dem Vorrastbereich befindliche Medium, als näherungsweise inkompressibel betrachtet werden, da in der Regel das Medium einen Kompressionsmodul aufweist, welcher derart ausgestaltet ist, dass die aufzuwendende Kraft für eine Volumenverringerung des Mediums höher ist, als die elastische Kraft, welche zwischen dem ersten Stopfen und der Innenwand des Spritzenkörpers wirkt. Es wird somit durch das Medium die Krafteinwirkung auf den ersten Stopfen übertragen, wodurch dieser sich in distaler Richtung (X1) verlagert.

Eine vorteilhafte Anwendung der Spritze kann demnach wie folgt ablaufen: Durch die Verlagerung des ersten Stopfens in den distalen Beabstandungsbereich wird die sich in dem distalen Beabstandungsbereich befindliche Luft durch den ersten Stopfen verdrängt. Ferner wird durch diese Verlagerung der zweite Übergangsbereich des Bypasskanals geöffnet und das Medium tritt in den Bypasskanal ein. Die auf den ersten Stopfen wirkende Kraft reduziert sich demnach entsprechend. Das Medium tritt durch den ersten Übergangsbereich in den geringen Restanteil des distalen Beabstandungsbereichs, der noch nicht durch den Stopfen ausgefüllt ist, in den Übergangsbereich des Spritzenkörpers und in den Durchtrittskanal ein. Auch aus diesen Bereichen wird die darin enthaltene Luft verdrängt. Durch weitere Kraftaufwendung auf den zweiten Stopfen wird das Medium vollständig aus dem Vorratsbereich gedrängt und gelangt über den Bypasskanal in den Durchtrittskanal und kann aus diesem durch die Austrittsöffnung austreten. Nachdem das Medium vollständig aus dem Vorratsraum verdrängt wurde, lieg vorteilhafterweise der zweite Stopfen an dem ersten Stopfen an. Vorzugsweise können nun beide Stopfen gemeinsam verlagert werden, wodurch der Rest an Medium aus dem Restanteil des distalen Beabstandungsbereichs und dem Übergangsbereich in den Durchtrittskanal gedrückt werden können.

Nach einer weiteren bevorzugten Ausführungsform ist der Flächeninhalt einer Querschnittsfläche des Bypasskanals zumindest 75%, bevorzugt zumindest 90% des Flächeninhalts einer Querschnittsfläche des Durchtrittskanals. Vorzugsweise ist der Flächeninhalt einer Querschnittsfläche des Bypasskanals höchstens 125%, bevorzugt höchstens 110% des Flächeninhalts einer Querschnittsfläche des Durchtrittskanals. Vorzugsweise ist der Flächeninhalt einer Querschnittsfläche des Bypasskanals im Wesentlichen gleich dem Flächeninhalt einer Querschnittsfläche des Durchtrittskanals. Bei dem zur Verabreichung vorgesehen Medium kann näherungsweise von einer inkompressiblen Flüssigkeit ausgegangen werden. Gemäß dem Kontinuitätsgesetz ist der Volumenstrom Q eine Erhaltungsgröße:

$$Q = A_1 \cdot v_1 = A_2 \cdot v_2$$

Bei einem sich verändernden Flächeninhalt ($A_1$, $A_2$) der Querschnittsfläche ändert sich die Volumengeschwindigkeit ($v_1$, $v_2$) entsprechend. Durch die genannten bevorzugten Ausgestaltungen ändert sich die Austrittsgeschwindigkeit des Mediums aus der Spritze im Vergleich zu bekannten Spritzen nur unwesentlich.

Nach einer weiteren bevorzugten Ausführungsform weist der erste Stopfen einen distalen Endbereich auf, welcher sich in distaler Richtung verjüngt. Eine solche Verjüngung kann beispielsweise der Form des Übergangsbereichs des Spritzenkörpers angepasst sein. Möglich wäre auch eine kegelartige Ausgestaltung des distalen Endbereichs, beispielsweise eines Vollkegels oder eines Kegelstumpfs. Durch eine solche Ausgestaltung wird es ermöglicht, dass die sich in dem Übergangsbereich befindliche Restmenge an Medium möglichst voll-ständig aus diesem in den Durchtrittskanal befördert werden kann.

Weiterhin kann es von Vorteil sein, wenn der erste Stopfen an einem proximalen Ende eine proximale Endfläche aufweist, welche planar ausgebildet ist. Durch eine solch planare Ausgestaltung und einer vorteilhaften planaren Ausgestaltung der distalen Endfläche des zweiten Stopfens, kann das Medium möglichst vollständig aus dem Vorratsbereich gedrängt werden. Die beiden Stopfen können nach dem Verdrängen des Mediums flächig anliegen.

Vorzugsweise besteht zumindest ein Mantelbereich des ersten Stopfens aus einem elastischen Material, so dass der erste Stopfen dichtend an einer Innenwand des hohlzylindrischen Spritzenkörpers anliegt. Der erste Stopfen könnte somit vollständig aus einem elastischen Material sein oder einen nichtleastischen Kern aufweisen, welcher von einem Mantelbereich aus einem elastischen Material umgeben ist. Dieser Mantelbereich kann vorteilhafterweise lediglich einen axialen Bereich des ersten Stopfens umfassen. Demnach würde an dem sich verjüngenden distalen Endabschnitt kein Mantelbereich aus einem elastischen Material vorgesehen sein. Natürlich könnte aus an diesem distalen Endabschnitt Mantelbereich aus einem elastischen Material vorgesehen sein. Vorteilhafterweise wird beim Einbringen des zweiten Stopfens in den Hohlraum des Spritzenkörpers das elastische Material komprimiert, so dass der zweite Stopfen dichtend an einer Innenwand des hohlzylindrischen Spritzenkörpers anliegt.

Nach einer weiteren bevorzugten Ausführungsform ist das hohlzylindrische Endstück als ein Konus ausgebildet, dessen Außendurchmesser sich kontinuierlich verjüngt. Vorzugsweise ist, das hohlzylindrische Endstück als Luer-Konus ausgebildet. Ein Luer-Ansatz-, Luer-Steck- oder Luer-Slip-System ist ein genormtes (ISO-Norm 594) Verbindungssystem für Schlauchsysteme im medizinischen Bereich und garantiert die Kompatibilität zwischen verschiedenen Herstellern. Es findet unter anderem Anwendung bei Spritzen, Kanülen und Infusions-Schläuchen. Ein männlicher Luer-Konus ist ein flacher (Steigung 6%) Hohlkegel mit relativ großer Oberfläche und daher guter Haftung mit einem aufgesteckten (weiblichen) Luer-Ansatz von beispielsweise einer Kanüle. Durch die kegelförmige Konstruktion beider Verbindungsteile wird eine ausreichende Dichtung erreicht.

Gemäß einer weiteren bevorzugten Ausführungsform ist an dem distalen Ende des Spritzenkörpers eine Überwurfmutter mit einem Innengewinde angeordnet, wobei das Endstück zentrisch von der Überwurfmutter umgeben ist. Durch eine derartige Anordnung kann eine Verbindung zwischen der vorgefüllten Spritze und beispielsweise einem Schlauchsystem verriegelt und gegen ein versehentliches Lösen gesichert werden. Vorzugsweise sind die Abmessungen des Endstücks und der Überwurfmutter genormt, so dass ein sogenanntes Luer-Lock-System realisiert wird.

Nach einer weiteren bevorzugten Ausführungsform ist ein Stechmittel in dem Durchtrittskanal des Endstücks befestigt. Vorteilhafterweise ist das Stechmittel mittels einer stoffschlüssigen Verbindung verbunden. Bevorzugt ist diese stoffschlüssige Verbindung eine Klebeverbindung. Das Stechmittel wird hierbei vorzugsweise zunächst in das distale Endstück der Spritze eingebracht und anschließend mit einem Kleber in dem Endabschnitt fixiert. Üblicherweise wird ein organischer Klebstoff verwendet, der mit UV-Licht aushärtbar ist. Derartige Spritzen werden auch oft als sogenannte "staked in needle" (SIN) Spritzen bezeichnet.

Da bei SIN Spritzen die Kanüle in dem Kanal befestigt bzw. verklebt wird, ist im Vergleich zu Luer-Konus Spritzen bei diesen SIN Spritzen oft ein Kanal geringerem Durchmesser erforderlich. Dabei ist es vorteilhaft, wenn der Durchtrittskanal in dem distalen Abschnitt einen größeren Innendurchmesser als in dem proximalen Abschnitt aufweist. In diesem distalen Abschnitt wird das Stechmittel eingesetzt und befestigt bzw. verklebt. Der Übergangsbereich zu dem proximalen Abschnitt mit dem kleineren Durchmesser kann als Anlagefläche für das Stechmittel dienen. Demnach ist es möglich, Spritzen mit einer konstanten effektiven Stechmittellänge herzustellen. Bevorzugt liegt der Durchmesser des distalen Abschnitts in einem Bereich zwischen 0,7 und 1,3 mm, besonders bevorzugt bei etwa 1 mm. Der Durchmesser des proximalen Abschnitts liegt bevorzugt in einem Bereich zwischen 0,45 mm und 0,9 mm und besonders bevorzugt bei etwa 0,7 mm.

Nach einer weiteren bevorzugten Ausführungsform ist der Spritzenkörper aus Glas gefertigt. Vorzugsweise besteht der Spritzenkörper aus Silikatglas, beispielsweise aus Borosilikatglas oder aus Quarzglas. Derartige Glasspritzen weisen eine hervorragende chemische Resistenz, Neutralität und Undurchlässigkeit auf.

In einer bevorzugten Ausführungsform besteht der Spritzenkörper aus einem Polymer-Kunststoff, bevorzugt aus einem Polyolefin, beispielsweise Polypropylen oder Polyethylen, besonders bevorzugt aus einem Cyclo-Olefinen Polymer (COP) beziehungsweise aus einem Cyclo-Olefinen Co-Polymer (COC). COC ist im Gegensatz zu den teilkristallinen Polyolefinen, wie Polyethylen und Polypropylen, amorph und damit transparent. Derartige Kunststoffe zeichnen sich dadurch aus, dass sie eine hervorragende Biokompatibilität, insbesondere Blutverträglichkeit sowie eine äußerst geringe Wasseraufnahme/ Wasserdampfdurchlässigkeit aufweisen. Weiterhin zeigen diese Kunststoffe keine Reaktion mit den üblichen zur Anwendung kommenden Medikamenten. Ein weiterer Vorteil von Kunststoffspritzen ist, dass auch bei spezifischen Anwendungen (z. B. Applikation unter der Zunge bei Krampfanfällen oder bei Veterinär-Anwendungen mit unruhigen Tieren) die Applikatorspitze für den Anwendungsfall abgestimmt, einteilig am Spritzenkörper ausgeführt werden kann, d.h. es werden keine zusätzlichen Bauteile/ Bedienungsvorgänge benötigt.

Die der Erfindung zugrundeliegende Aufgabe wird auch durch ein Spritze gemäß einer der vorhergehenden Ausführungsformen gelöst, wobei in einem Ruhezustand der Vorratsbereich mit dem zur Verabreichung vorgesehene Medium befüllt ist und in dem distalen Beabstandungsbereich kein zur Verabreichung vorgesehene Medium vorhanden ist.

Schließlich wird die Aufgabe durch eine Verwendung einer vorfüllbaren Spritze nach einer der vorhergehenden Ausführungsformen gelöst, wobei in einem Ruhezustand der Vorratsbereich mit dem zur Verabreichung vorgesehene Medium befüllt wird und in dem distalen Beabstandungsbereich kein zur Verabreichung vorgesehenes Medium vorhanden ist.

Eine vorteilhafte Anwendung der Spritze kann demnach wie folgt ablaufen: Durch die Verlagerung des ersten Stopfens in den distalen Beabstandungsbereich wird die sich in dem distalen Beabstandungsbereich befindliche Luft durch den ersten Stopfen verdrängt. Ferner wird durch diese Verlagerung der zweite Übergangsbereich des Bypasskanals geöffnet und das Medium tritt in den Bypasskanal ein. Die auf den ersten Stopfen wirkende Kraft reduziert sich demnach entsprechend. Das Medium tritt durch den ersten Übergangsbereich in den geringen Restanteil des distalen Beabstandungsbereichs, der noch nicht durch den Stopfen ausgefüllt ist, in den Übergangsbereich des Spritzenkörpers und in den Durchtrittskanal ein. Auch aus diesen Bereichen wird die darin enthaltene Luft verdrängt. Durch weitere Kraftaufwendung auf den zweiten Stopfen wird das Medium vollständig aus dem Vorratsbereich gedrängt und gelangt über den Bypasskanal in den Durchtrittskanal und kann aus diesem durch die Austrittsöffnung austreten. Nachdem das Medium vollständig aus dem Vorratsraum verdrängt wurde, lieg vorteilhafterweise der zweite Stopfen an dem ersten Stopfen an. Vorzugsweise können nun beide Stopfen gemeinsam verlagert werden, wodurch der Rest an Medium aus dem Restanteil des distalen Beabstandungsbereichs und dem Übergangsbereich in den Durchtrittskanal gedrückt werden können.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:

Fig.1       eine Schnittdarstellung einer vorfüllbaren Spritze nach einer Ausführungsform;

Fig.2       eine Außendarstellung einer vorfüllbaren Spritze nach einer Ausführungsform;

Fig.3       eine Schnittdarstellung einer vorfüllbaren Spritze nach einer Ausführungsform;

Fig.4       eine Schnittdarstellung einer vorfüllbaren Spritze nach einer Ausführungsform;

Fig.5       eine Schnittdarstellung einer vorfüllbaren Spritze nach einer Ausführungsform;

Fig. 6       eine Schnittdarstellung eines ersten Stopfens nach einer Ausführungsform;

Fig. 7       eine Schnittdarstellung eines zweiten Stopfens nach einer Ausführungsform;

Fig.8a) - d)       eine Schnittdarstellung einer vorfüllbaren Spritzen nach weiteren Ausführungsformen;

Fig.9a) - d)       Darstellungen von vorfüllbaren Spritzen nach verschiedenen Ausführungsformen;

Fig.10a) - b)       Darstellungen von vorfüllbaren Spritzen nach verschiedenen Ausführungsformen;

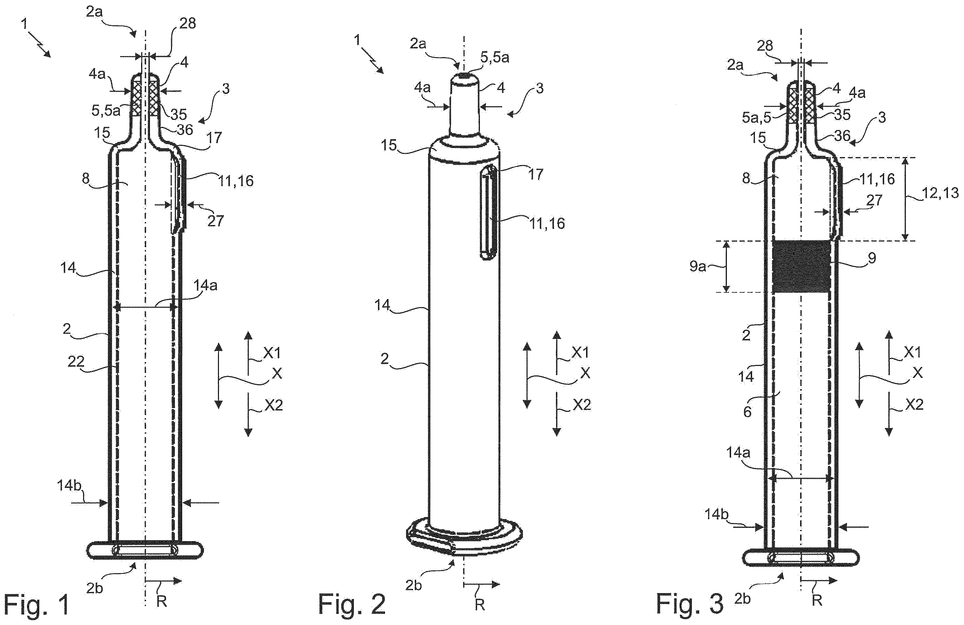

Die Figuren 2 bis 5 und 7 bis 10 zeigen eine vorfüllbare Spritze (1) für medizinische Anwendungen mit einen hohlzylindrischen sich entlang einer axialen Achse (X) erstreckenden Spritzenkörper (2), an dessen distalem Ende (3) ein hohlzylindrisches Endstück (4) ausgeformt ist, welches einen Durchtrittskanal (5) aufweist, wobei der Spritzenkörper (2) einen Vorratsbereich (6) für ein zur Verabreichung vorgesehenes Medium (7) umfasst, wobei in einem Ruhezustand der Vorratsbereich (6) durch einen distalen Beabstandungsbereich (8) von dem Durchtrittskanal (5) des hohlzylindrischen Endstücks (4) entlang der axialen Achse (X) beabstandet ist und ein erster Stopfen (9) den Vorratsbereich (6) von dem distalen Beabstandungsbereich (8) trennt, wobei in einem Abgabezustand durch eine Verlagerung des ersten Stopfens (9) in den distalen Beabstandungsbereich (8) eine fluidische Verbindung (10) für das Medium (7) zwischen dem Vorratsbereich (6) und dem Durchtrittskanal (5) über einen Bypasskanal (11) öffenbar ist, wobei sich eine axiale Länge des Bypasskanals (12) sich zumindest über eine axiale Länge (13) des distalen Beabstandungsbereichs (8) erstreckt.

Figur 1 zeigt einen Spritzenkörper (2), welcher mit einem Bypasskanal (11) versehen ist, jedoch ist in diesem Spritzenkörper noch kein erster Stopfen (9) eingebracht.

Der Spritzenkörper (2) ist in drei Abschnitte unterteilbar, wobei der Spritzenkörper einstückig ausgebildet ist. Zunächst besteht der Spritzenkörper (2) aus einem Hauptabschnitt (14), welcher bevorzugt als ein Hohlkörper mit kreisförmiger Grundfläche ausgebildet ist. In diesem Hauptabschnitt (14) ist der Innendurchmesser des Hohlkörpers außer im Bereich des Bypasskanals im Wesentlichen konstant. In distaler Richtung (X1) läuft der Spritzenkörper (2) in ein hohlzylindrisches Endstück (4) aus. Das Endstück (4) hat einen Durchtrittskanal (5) dessen Durchmesser (5a) kleiner ist als der Innendurchmesser (14a) des Hauptabschnitts (14).

Weiterhin ist ein Übergangsbereich (15) zwischen dem Hauptabschnitt (14) und dem Endstück (4) vorgesehen. Der distale Beabstandungsbereich (8) ist dabei ein Teil des Hauptabschnitts (14) und schließt an dem Übergangsbereich (15) an.

Ein Außendurchmesser (14b) des Hauptabschnitts (14) ist größer als ein Außendurchmesser (4a) des Endstücks (4), wobei bevorzugt der Außendurchmesser (14b) des Hauptabschnitts (14) über seine gesamte axiale Länge, ausgenommen im Bereich des Bypasses konstant ist. In dem Übergangsbereich (15) findet demnach ein Übergang von dem größeren Außendurchmesser (14b) des Hauptabschnitts (14) auf den kleineren Außendurchmesser (4a) des Endstücks (4) statt. Ebenfalls findet in dem Übergangsbereich (15) ein Übergang von dem größeren Innendurchmesser (14a) des Hauptabschnitts (14) zu dem kleineren Durchmesser (5a) des Durchtrittskanals (5) statt.

An dem proximalen Ende des Hauptabschnitts (14) ist eine Fingerauflage (34) vorgesehen. Ferner ist an dem proximalen Ende des Spritzenkörpers eine proximale Öffnung (2b) des Spritzenkörpers (2) vorgesehen. An dem distalen Ende des Endstücks (4) ist weiterhin eine distale Öffnung (2a) vorgesehen, in welche der Durchtrittskanal (5) mündet.

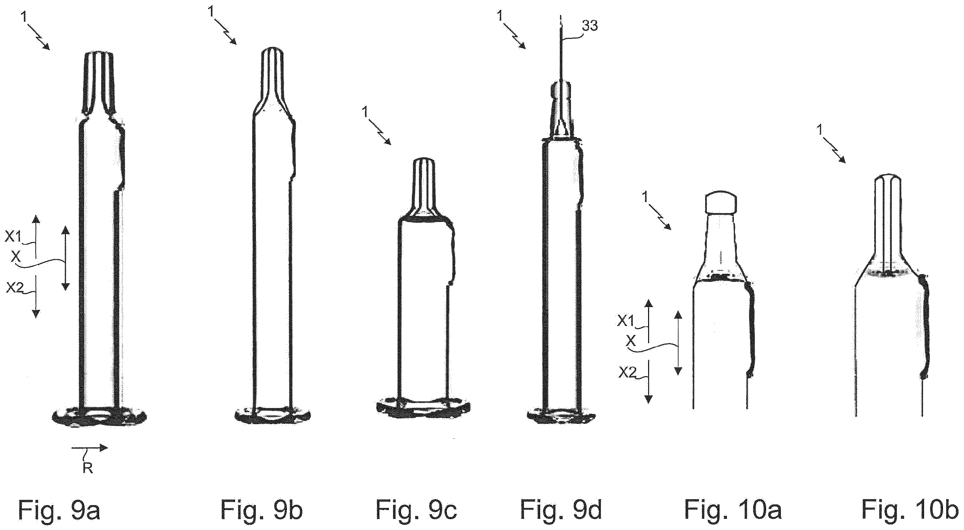

Das hohlzylindrische Endstück (4) ist als ein Konus ausgebildet, dessen Außendurchmesser sich kontinuierlich verjüngt. Vorzugsweise kann das hohlzylindrische Endstück als ein Luer-Konus ausgebildet sein. Dies ist beispielsweise in Figur 10b erkennbar.

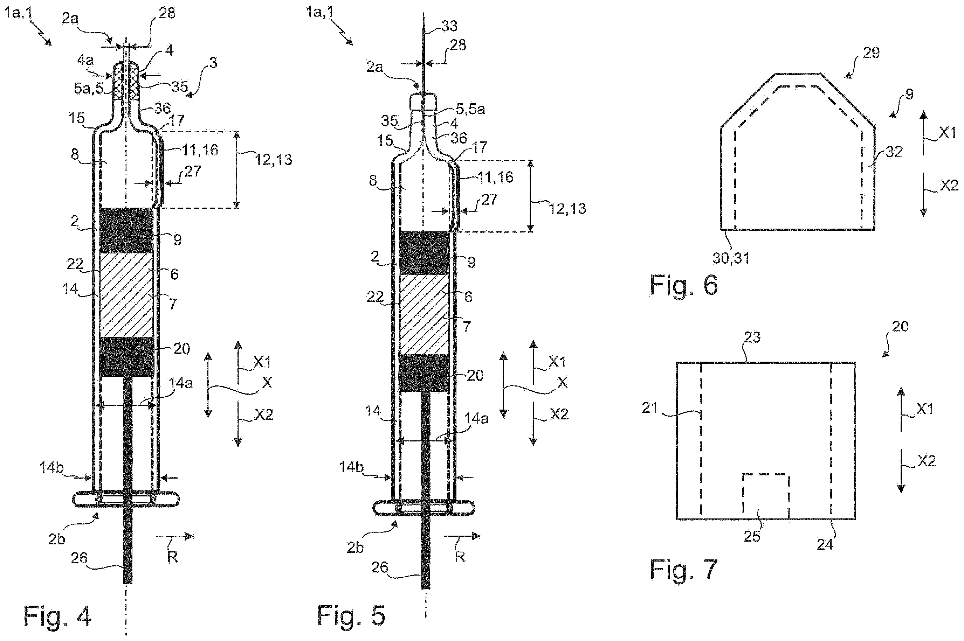

Gemäß der Ausführungsform nach den Figur 5 und 9d ist ein Stechmittel (33) in dem Durchtrittskanal (5) des Endstücks (4) befestigt. Vorteilhafterweise ist das Stechmittel (33) mittels einer stoffschlüssigen Verbindung insbesondere einer Klebeverbindung mit dem Endstück (4) verbunden. Ein Endstück (4), welches für das Einführen eines Stechmittels (33) vorgesehen ist, ist beispielsweise in Figur 10a erkennbar.

Der Durchtrittskanal (5) kann einen distalen Abschnitt (35) aufweisen, in welchem das Stechmittel (33) mit dem Durchtrittskanal verklebt ist. Dieser Durchtrittskanal kann vorteilhafterweise einen größeren Innendurchmesser als in dem proximalen Abschnitt (36) des Durchtrittskanals aufweisen. Der Übergangsbereich zu dem proximalen Abschnitt (36) mit dem kleineren Durchmesser kann als Anlagefläche für das Stechmittel (33) dienen. Demnach ist es möglich, Spritzen mit einer konstanten effektiven Stechmittellänge herstellen.

Der Bypasskanal (11) ist eine in radialer Richtung (R) nach außen gerichtete Auswölbung (16) des Spritzenkörpers (2). Ein distales Ende (17) des Bypasskanals (11) grenzt im Wesentlichen an dem Übergangsbereich (15) an, d. h. es ist lediglich ein geringer herstellungsbedingter Toleranzabstand zwischen dem Übergangsbereich (15) beziehungsweise der Wölbung in diesem Bereich und dem distalen Ende (17) des Bypasskanals (11) vorhanden.

Der Bypasskanal (11) ist somit in der vorderen distalen Hälfte bevorzugt im vorderen Drittel, weiter bevorzugt im vorderen Viertel des Hauptabschnitts (14) angeordnet.

Die axiale Länge (13) des distalen Beabstandungsbereichs (8) entspricht einer axialen Länge (18) des ersten Stopfens (9) und einer Länge eines ersten (18) und eines zweiten Übergriffsbereichs (19) des Bypasskanals (11).

Der Flächeninhalt einer Querschnittsfläche (27) des Bypasskanals (11) ist zumindest 75%, bevorzugt zumindest 90% des Flächeninhalts einer Querschnittsfläche (28) des Durchtrittskanals. Der Flächeninhalt einer Querschnittsfläche (27) des Bypasskanals (11) ist höchstens 125%, bevorzugt höchstens 110% des Flächeninhalts einer Querschnittsfläche (28) des Durchtrittskanals (5). Vorteilhafterweise ist der Flächeninhalt der Querschnittsfläche (28) des Bypasskanals (11) im Wesentlichen gleich dem Flächeninhalt der Querschnittsfläche (28) des Durchtrittskanals (5).

Der Vorratsbereich ist durch einen zweiten Stopfen (20) in proximaler Richtung (X2) begrenzt. Ein entsprechender zweiter Stopfen (20) ist in Figur 7 gezeigt und weist zumindest einen Mantelbereich auf, welcher aus einem elastischen Material besteht, so dass der zweite Stopfen (20) dichtend an einer Innenwand (22) des hohlzylindrischen Spritzenkörpers (2) anliegt. Der zweite Stopfen weist weiterhin an einem distalen Ende eine distale Endfläche (23) auf, welche planar ausgebildet ist. Ferner umfasst der zweite Stopfen an einem proximalen Ende eine Sackbohrung (25) mit einem Innengewinde aufweist, in welchem eine Kolbenstange (26) anordenbar ist.

Durch eine Verlagerung des zweiten Stopfens (20) in dem Spritzenkörper (2) entlang der in distaler Richtung (X1), ist die Verlagerung des ersten Stopfens (9) in den distalen Beabstandungsbereich (8) hervorrufbar.

Der erste Stopfen (9) ist in Fig 6 dargestellt und weist einen distalen Endbereich (29), welcher sich in distaler Richtung (X1) verjüngt. Eine solche Verjüngung kann beispielsweise der Form des Übergangsbereichs (15) des Spritzenkörpers (2) angepasst sein. Möglich wäre auch eine kegelartige Ausgestaltung des distalen Endbereichs (29), beispielsweise eines Vollkegels oder eines Kegelstumpfs. Der erste Stopfen (9) weist an einem proximalen Ende (30) eine proximale Endfläche (31) auf, welche planar ausgebildet ist. Ferner umfasst der erste Stopfen (9) einen Mantelbereich (32), welcher aus einem elastischen Material besteht, so dass der erste Stopfen (9) dichtend an einer Innenwand (22) des hohlzylindrischen Spritzenkörpers (2) anliegt.

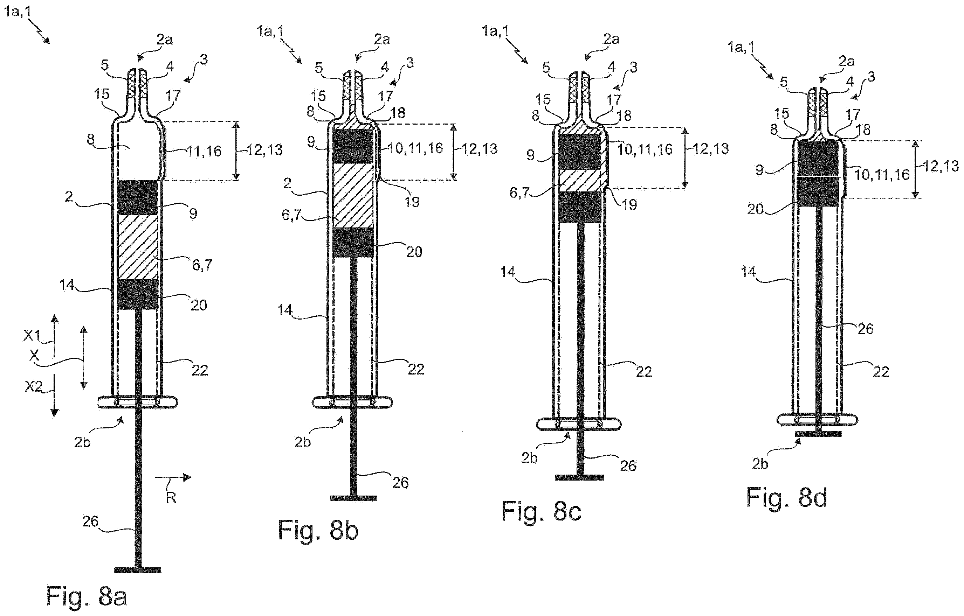

In den Figuren 8a bis d ist der Ablauf einer Anwendung einer vorfüllbaren Spritze dargestellt. In Figur 8a ist der Ruhezustand dargestellt. Der Vorratsbereich (6) ist durch einen distalen Beabstandungsbereich (8) von dem Durchtrittskanal (5) des hohlzylindrischen Endstücks (4) entlang der axialen Achse (X) beabstandet, wobei ein erster Stopfen (9) den Vorratsbereich (6) von dem distalen Beabstandungsbereich (8) trennt. In einem Abgabezustand gemäß den Figuren 8a bis d ist durch eine Verlagerung des ersten Stopfens (9) in den distalen Beabstandungsbereich (8) eine fluidische Verbindung (10) für das Medium (7) zwischen dem Vorratsbereich (6) und dem Durchtrittskanal (5) über einen Bypasskanal (11) öffenbar, wobei sich eine axiale Länge des Bypasskanals (12) zumindest über eine axiale Länge (13) des distalen Beabstandungsbereichs (8) erstreckt.

Die Verlagerung des ersten Stopfens (9) in den distalen Beabstandungsbereich (8) ist durch eine Verlagerung des zweiten Stopfens (20) in distaler Richtung (X1) hervorrufbar. In dem Abgabezustand wird demnach eine Kraft, vorzugsweise durch eine Kolbenstange (26), auf den zweiten Stopfen (20) ausgeübt, so dass dieser sich in distaler Richtung (X1) verlagert. Diese Kraft wird durch das sich in dem Vorratsbereich (8) befindliche Medium (7) auf den ersten Stopfen (9) übertragen, wodurch dieser sich in distaler Richtung (X1) verlagert.

Durch die Verlagerung des ersten Stopfens (9) in den distalen Beabstandungsbereich (8) wird die sich in dem distalen Beabstandungsbereich (8) befindliche Luft durch den ersten Stopfen (9) verdrängt. Ferner wird durch diese Verlagerung der zweite Übergangsbereich (19) des Bypasskanals (11) geöffnet und das Medium (7) tritt in den Bypasskanal (11) ein. Dies ist in Figur 8b) ersichtlich. Die auf den ersten Stopfen (9) wirkende Kraft reduziert sich demnach entsprechend. Aus dem Bypasskanal (11) tritt das Medium (7) durch den ersten Übergangsbereich (18) in den geringen Restanteil des distalen Beabstandungsbereichs (8), der noch nicht durch den ersten Stopfen (9) ausgefüllt ist. Weiter tritt das Medium (7) in den Übergangsbereich (15) des Spritzenkörpers (2) und in den Durchtrittskanal (5) ein. Auch aus diesen Bereichen wird somit die darin enthaltene Luft verdrängt.

Durch weitere Kraftaufwendung auf den zweiten Stopfen (20) wird das Medium (7) durch den Bypasskanal (11) und den Durchtrittskanal der distalen Öffnung (2a) beziehungsweise dem Stechmittel (33) zugeführt. Das Medium (7) wird schließlich vollständig aus dem Vorratsbereich (6) gedrängt und gelangt über den Bypasskanal (11) in den Durchtrittskanal (5). Durch eine vorteilhafte Ausgestaltung einer planaren distale Endfläche (23) des zweiten Stopfens (20) und einer planaren proximalen Endfläche (31) des ersten Stopfens kann das Medium (7) möglichst vollständig aus dem Vorratsbereich (6) gedrängt werden. Die beiden Stopfen (9, 20) können nach dem Verdrängen des Mediums (7) flächig anliegen. Beide Stopfen (9, 20) können nun gemeinsam verlagert werden, wodurch der Rest an Medium (7) aus dem Restanteil des distalen Beabstandungsbereichs (8) und dem Übergangsbereich (15) in den Durchtrittskanal gedrückt werden können. Durch eine vorteilhafte Ausgestaltung des ersten Stopfens (9) mit einem sich verjüngenden distalen Endbereich (29) wird es ermöglicht, dass die sich die in dem Übergangsbereich (15) befindliche Restmenge an Medium (7) möglichst voll-ständig aus diesem in den Durchtrittskanal (5) befördert werden kann.

In den Figuren 9a bis 9d sind mögliche Ausführungsformen der vorfüllbaren Spritze mit einem Volumen von 0,5 ml (Fig. 9a) von 1,0ml (Fig. 9b) und 5ml (Fig. 9c). Figur 9d zeigt weiterhin eine vorfüllbare Spritze mit einem Volumen von 1,0 ml, welche mit einem Stechmittel (33) versehen ist.

Sämtliche in den Anmeldungsunterlagen offenbarten Merkmale werden als erfindungswesentlich beansprucht, sofern sie einzeln oder in Kombination gegenüber dem Stand der Technik neu sind.

**Bezugszeichenliste**

| | |
|---|---|
| 1 | Spritze |
| 1a | Spritze (befüllt) |
| 2 | Spritzenkörper |
| 2a | distale Öffnung des Spritzenkörpers |
| 2b | proximale Öffnung des Spritzenkörpers |
| 3 | distales Ende |
| 4 | Endstück |
| 4a | Außendurchmesser des Endstücks |
| 5 | Durchtrittskanal |
| 5a | Durchmesser des Durchtrittskanals |
| 6 | Vorratsbereich |
| 7 | zur Verabreichung vorgesehenes Medium |
| 8 | distaler Beabstandungsbereich |
| 9 | erster Stopfen |
| 10 | fluidische Verbindung |
| 11 | Bypasskanal |
| 12 | axiale Länge des Bypasskanals |
| 13 | axiale Länge des distalen Beabstandungsbereichs |
| 14 | Hauptabschnitt des Spritzenkörpers |
| 14a | Innendurchmesser des Hauptabschnitts |
| 14b | Außendurchmesser des Hauptabschnitts |
| 15 | Übergangsbereich des Spritzenkörpers |
| 16 | Auswölbung des Spritzenkörpers |
| 17 | distales Ende des Bypasskanals |
| 18 | erster Übergriffsbereich |
| 19 | zweiter Übergriffsbereich |
| 20 | distaler Abschnitt des Kanals |
| 21 | Mantelbereich des zweiten Stopfens |
| 22 | Innenwand des Spritzenkörpers |
| 23 | distale Endfläche des zweiten Stopfens |
| 24 | proximales Ende des zweiten Stopfens |

25    Sackbohrung
26    Kolbenstange
27    Querschnittsfläche des Bypasskanals
28    Querschnittsfläche des Durchtrittskanals
29    distalen Endbereich des ersten Stopfens
30    proximalen Ende des ersten Stopfens
31    proximale Endfläche des ersten Stopfens
32    Mantelbereich des ersten Stopfens
33    Stechmittel
34    Fingerauflage
35    Klebebereich
X     axiale Achse
X1    distaler Abschnitt des Durchtrittskanals
X2    proximale Richtung
R     radiale Richtung

**Patentansprüche**

1.   Spritze (1a) für medizinische Anwendungen mit einen hohlzylindrischen, sich entlang einer axialen Achse (X) erstreckenden Spritzenkörper (2), an dessen distalem Ende (3) ein hohlzylindrisches Endstück (4) ausgeformt ist, welches einen Durchtrittskanal (5) aufweist, wobei der Spritzenkörper (2) einen Vorratsbereich (6) für ein zur Verabreichung vorgesehenes Medium (7) umfasst, wobei in einem Ruhezustand der Vorratsbereich (6) durch einen distalen Beabstandungsbereich (8) von dem Durchtrittskanal (5) des hohlzylindrischen Endstücks (4) entlang der axialen Achse (X) beabstandet ist und ein erster Stopfen (9) den Vorratsbereich (6) von dem distalen Beabstandungsbereich (8) trennt, wobei in einem Abgabezustand durch eine Verlagerung des ersten Stopfens (9) in den distalen Beabstandungsbereich (8) eine fluidische Verbindung (10) für das Medium (7) zwischen dem Vorratsbereich (6) und dem Durchtrittskanal (5) über einen Bypasskanal (11) öffenbar ist, wobei sich eine axiale Länge des Bypasskanals (12) sich zumindest über eine axiale Länge (13) des distalen Beabstandungsbereichs (8) erstreckt, **dadurch gekennzeichnet, dass** in einem Ruhezustand vor Anwendung der Spritze der Vorratsbereich (6) mit dem zur Verabreichung vorgesehenen Medium (7) befüllt ist und in dem distalen Beabstandungsbereich (8) kein zur Verabreichung vorgesehenes Medium vorhanden ist.

2.   Spritze (1a) nach Anspruch 1,
     **dadurch gekennzeichnet, dass**
     der Spritzenkörper (2) einen Hauptabschnitt (14) aufweist, welcher als ein Hohlkörper mit kreisförmiger Grundfläche ausgebildet ist, wobei ein Übergangsbereich (15) zwischen dem Hauptabschnitt (14) und dem Endstück (4) vorgesehen ist, wobei der distale Beabstandungsbereich (8) ein Teil des Hauptabschnitts (14) ist und an dem Übergangsbereich (15) anschließt.

3.   Spritze (1a) nach Anspruch 1 oder 2,
     **dadurch gekennzeichnet, dass**.
     der Bypasskanal (11) eine in radialer Richtung (R) nach außen gerichtete Auswölbung (16) des Spritzenkörpers (2) ist, wobei ein distales Ende (17) des Bypasskanals (11) im Wesentlichen an dem Übergangsbereich (15) angrenzt.

4.   Spritze (1a) nach einem der vorhergehenden Ansprüche,
     **dadurch gekennzeichnet, dass**.
     die axiale Länge (13) des distalen Beabstandungsbereichs (8) einer axialen Länge (18) des ersten Stopfens (9) und einer Länge eines ersten (18) und eines zweiten Übergriffbereichs (19) des Bypasskanals (11) entspricht.

5.   Spritze (1a) nach einem der vorhergehenden Ansprüche,
     **dadurch gekennzeichnet, dass**
     der Vorratsbereich (6) durch einen zweiten Stopfen (20) in proximaler Richtung (X2) begrenzt ist, wobei zumindest ein Mantelbereich (21) des zweiten Stopfens (20) aus einem elastischen Material besteht, so dass der zweite Stopfen (20) dichtend an einer Innenwand (22) des Spritzenkörpers (2) anliegt, wobei der zweite Stopfen (20) eine distale Endfläche (23) aufweist, welche planar ausgebildet ist, wobei der zweite Stopfen (20) an einem proximalen Ende eine Sackbohrung (25) mit einem Innengewinde aufweist, in welchem eine Kolbenstange (26) anordenbar ist.

6.   Spritze (1a) nach einem der vorhergehenden Ansprüche,
     **dadurch gekennzeichnet, dass**
     der zweite Stopfen (20) in dem Spritzenkörper (2) entlang der axialen Achse (X) verlagerbar ist, wobei durch eine Verlagerung des zweiten Stopfens in distaler Richtung (X1) die Verlagerung des ersten Stopfens (9) in den distalen Beabstandungsbereich (8) hervorrufbar ist.

7.   Spritze (1a) nach einem der vorhergehenden Ansprüche,
     **dadurch gekennzeichnet, dass**
     der Flächeninhalt einer Querschnittsfläche (27) des Bypasskanals (11) zumindest 75% bevorzugt zumindest 90% des Flächeninhalts einer Querschnittsfläche (28) des Durchtrittskanals ist, wobei der Flächeninhalt einer Querschnittsfläche (27) des Bypasskanals (11) höchstens 125% bevorzugt höchstens 110% des Flächeninhalts einer Querschnittsfläche (28) des Durchtrittskanals (5) ist, wobei der Flächeninhalt einer Querschnittsfläche (28) des Bypasskanals (11) im Wesentlichen gleich dem Flächeninhalts einer Querschnittsfläche (28) des Durchtrittskanals (5) ist.

8. Spritze (1a) nach einem der vorhergehenden Ansprüche,
**dadurch gekennzeichnet, dass**
der erste Stopfen (9) einen distalen Endbereich (29) aufweist, welcher sich in distaler Richtung (X1) verjüngt, wobei der erste Stopfen (9) an einem proximalen Ende (30) eine proximale Endfläche (31) aufweist, welche planar ausgebildet ist, wobei zumindest ein Mantelbereich (32) des ersten Stopfens (9) aus einem elastischen Material besteht, so dass der erste Stopfen (9) dichtend an einer Innenwand (22) des hohlzylindrischen Spritzenkörpers (2) anliegt.

9. Spritze (1a) nach einem der vorhergehenden Ansprüche,
**dadurch gekennzeichnet, dass**..
das hohlzylindrische Endstück (4) als ein Konus ausgebildet ist, dessen Außendurchmesser sich kontinuierlich verjüngt, wobei das hohlzylindrische Endstück (4) als ein Luer-Konus ausgebildet ist.

10. Spritze (1a) nach einem der vorhergehenden Ansprüche,
**dadurch gekennzeichnet, dass**
ein Stechmittel (33) in dem Durchtrittskanal (5) des Endstücks (4) befestigt ist, wobei das Stechmittel (33) mittels einer stoffschlüssigen Verbindung insbesondere einer Klebeverbindung mit dem Endstücks (4) verbunden ist

11. Spritze (1a) nach einem der vorhergehenden Ansprüche,
**dadurch gekennzeichnet, dass**
der Spritzenkörper (2) aus Glas gefertigt ist, wobei der Spritzenkörper (2) aus Silikatglas besteht.

12. Verwendung einer Spritze (1a) nach einem der Ansprüche 1 bis 11 zur Vermeidung eines Kontakts des zur Verabreichung vorgesehenen Mediums (7) mit riskanten Kontaktmaterialien in der Spritze während der Lagerung der Spritze, wobei in einem Ruhezustand der Vorratsbereich (6) mit dem zur Verabreichung vorgesehene Medium (7) befüllt wird und in dem distalen Beabstandungsbereich (8) kein zur Verabreichung vorgesehenes Medium vorhanden ist.

**Claims**

1. Syringe (1a) for medical applications with a hollow cylindrical syringe body (2) extending along an axial axis (X), at the distal end (3) of which a hollow cylindrical end piece (4) is formed which has a passage channel (5), wherein the syringe body (2) comprises a storage area (6) for a medium (7) intended for administration, wherein
in a rest state, the storage area (6) is spaced from the passage channel (5) of the hollow cylindrical end piece (4) along the axial axis (X) by a distal spacing area (8) and a first plug (9) separates the storage area (6) from the distal spacing area (8), wherein, in a dispensing state, a fluidic connection (10) for the medium (7) between the storage area (6) and the passage channel (5) can be opened via a bypass channel (11) by displacing the first plug (9) into the distal spacing area (8), wherein an axial length of the bypass channel (12) extends at least over an axial length (13) of the distal spacing area (8), **characterized in that** in a rest state before use of the syringe the storage area (6) is filled with the medium (7) intended for administration and no medium intended for administration is present in the distal spacing area (8).

2. Syringe (1a) according to claim 1,
**characterized in that**
said syringe body (2) comprises a main portion (14) formed as a hollow body having a circular base, a transition area (15) being provided between said main portion (14) and said end piece (4), said distal spacing area (8) being a part of said main portion (14) and adjoining said transition area (15).

3. Syringe (1a) according to claim 1 or 2,
**characterised in that**.
the bypass channel (11) is an outwardly directed bulge (16) of the syringe body (2) in the radial direction (R), a distal end (17) of the bypass channel (11) being substantially adjacent to the transition area (15).

4. Syringe (1a) according to any one of the preceding claims,
**characterized in that**.
the axial length (13) of the distal spacing area (8) corresponds to an axial length (18) of the first plug (9) and to a length of a first (18) and a second overlap area (19) of the bypass channel (11).

5. Syringe (1a) according to any one of the preceding claims,
**characterized in that**
the storage area (6) is delimited by a second plug (20) in a proximal direction (X2), at least one jacket area (21) of the second plug (20) consisting of an elastic material, so that the second plug (20) bears sealingly against an inner wall (22) of the syringe body (2), wherein the second plug (20) has a distal end surface (23) which is planar, wherein the second plug (20) has at a proximal end a blind hole (25) with an internal thread in which a piston rod (26) can be arranged.

**6.** Syringe (1a) according to any one of the preceding claims,
**characterized in that**
the second plug (20) is displaceable in the syringe body (2) along the axial axis (X), wherein a displacement of the second plug in the distal direction (X1) can cause displacement of the first plug (9) into the distal spacing area (8).

**7.** Syringe (1a) according to any one of the preceding claims,
**characterized in that**
the surface area of a cross-sectional area (27) of the bypass channel (11) is at least 75% preferably at least 90% of the surface area of a cross-sectional area (28) of the passage channel, wherein the surface area of a cross-sectional surface (27) of the bypass channel (11) is at most 125% preferably at most 110% of the surface area of a cross-sectional surface (28) of the passage channel (5), wherein the surface area of a cross-sectional surface (28) of the bypass channel (11) is substantially equal to the surface area of a cross-sectional surface (28) of the passage channel (5).

**8.** Syringe (1a) according to any one of the preceding claims,
**characterized in that**
the first plug (9) has a distal end area (29) which tapers in the distal direction (X1), the first plug (9) having at a proximal end (30) a proximal end surface (31) which is planar, at least one jacket portion (32) of the first plug (9) consisting of an elastic material, so that the first plug (9) bears sealingly against an inner wall (22) of the hollow cylindrical syringe body (2).

**9.** Syringe (1a) according to any one of the preceding claims,
**characterised in that**.
the hollow cylindrical end piece (4) is formed as a cone whose outer diameter continuously tapers, wherein the hollow cylindrical end piece (4) is formed as a luer cone.

**10.** Syringe (1a) according to any one of the preceding claims,
**characterized in that**
a piercing means (33) is fixed in the passage channel (5) of the end piece (4), wherein the piercing means (33) is connected to the end piece (4) by means of a material connection, in particular an adhesive connection.

**11.** Syringe (1a) according to one of the preceding claims,
**characterised in that**
the syringe body (2) is made of glass, wherein the syringe body (2) is made of silicate glass.

**12.** Use of a syringe (1a) according to any one of claims 1 to 11 for avoiding contact of the medium (7) intended for administration with risky contact materials in the syringe during storage of the syringe, wherein in a rest state the storage area (6) is filled with the medium (7) intended for administration and no medium intended for administration is present in the distal spacing area (8).

**Revendications**

**1.** Seringue (1a) pour applications médicales comportant un corps de seringue (2) cylindrique creux, s'étendant le long d'un axe axial (X), à l'extrémité distale (3) duquel est formée une pièce d'extrémité cylindrique creuse (4), laquelle présente un canal de passage (5), le corps de seringue (2) comportant une zone de réserve (6) pour un milieu (7) prévu pour l'administration,
seringue selon laquelle, dans un état de repos, la zone de réserve (6) est espacée par une zone d'espacement distale (8) du canal de passage (5) de la pièce d'extrémité cylindrique creuse (4) le long de l'axe axial (X) et un premier bouchon (9) sépare la zone de réserve (6) de la zone d'espacement distale (8), seringue selon laquelle, dans un état de distribution par un déplacement du premier bouchon (9) dans la zone d'espacement distale (8), une liaison fluidique (10) pour le milieu (7) entre la zone de réserve (6) et le canal de passage (5) est apte à être ouverte par un canal de dérivation (11), une longueur axiale du canal de dérivation (12) s'étendant au moins sur une longueur axiale (13) de la zone d'espacement distale (8), **caractérisée par le fait que** dans un état de repos avant utilisation de la seringue, la zone de réserve (6) est remplie par le milieu (7) prévu pour l'administration et dans la zone d'espacement distale (8), aucun milieu prévu pour l'administration n'est présent.

**2.** Seringue (1a) selon la revendication 1, **caractérisée par le fait que**
le corps de seringue (2) présente une partie principale (14), laquelle est formée en tant qu'un corps creux ayant une base circulaire, une zone de transition (15) étant prévue entre la partie principale (14) et la pièce d'extrémité (4), la zone d'espacement distale (8) faisant partie de la partie principale (14) et étant contiguë à la zone de transition (15).

**3.** Seringue (1a) selon l'une des revendications 1 et 2, **caractérisée par le fait que**
le canal de dérivation (11) est un renflement (16) du corps de seringue (2) orienté vers l'extérieur dans la direction radiale (R), une extrémité distale (17) du canal de dérivation (11) étant sensiblement adjacente à la zone de transition (15).

4. Seringue (1a) selon l'une des revendications précédentes,
   **caractérisée par le fait que**
   la longueur axiale (13) de la zone d'espacement distale (8) correspond à une longueur axiale (18) du premier bouchon (9) et à une longueur d'une première (18) et d'une seconde (19) zone de chevauchement du canal de dérivation (11).

5. Seringue (1a) selon l'une des revendications précédentes,
   **caractérisée par le fait que**
   la zone de réserve (6) est limitée par un second bouchon (20) dans la direction proximale (X2), au moins une zone d'enveloppe (21) du second bouchon (20) étant constituée d'un matériau élastique, de telle sorte que le second bouchon (20) s'applique de manière étanche contre une paroi interne (22) du corps de seringue (2), le second bouchon (20) présentant une face d'extrémité distale (23), laquelle est conçue de manière plane, le second bouchon (20) présentant à une extrémité proximale un alésage borgne (25) ayant un filetage interne dans lequel une tige de piston (26) est apte à être disposée.

6. Seringue (1a) selon l'une quelconque des revendications précédentes,
   **caractérisée par le fait que**
   le second bouchon (20) est apte à être déplacé dans le corps de seringue (2) le long de l'axe axial (X), un déplacement du second bouchon dans la direction distale (X1) permettant de provoquer le déplacement du premier bouchon (9) dans la zone d'espacement distale (8).

7. Seringue (1a) selon l'une des revendications précédentes,
   **caractérisée par le fait que**
   l'aire de surface d'une surface de section transversale (27) du canal de dérivation (11) est d'au moins 75%, de préférence d'au moins 90% de l'aire de surface d'une surface de section transversale (28) du canal de passage, l'aire de surface d'une surface de section transversale (27) du canal de dérivation (11) étant d'au plus 125%, de préférence d'au plus 110%, de l'aire de surface d'une surface de section transversale (28) du canal de passage (5), l'aire de surface d'une surface de section transversale (28) du canal de dérivation (11) étant sensiblement égale à l'aire de surface d'une surface de section transversale (28) du canal de passage (5).

8. Seringue (1a) selon l'une des revendications précédentes,
   **caractérisée par le fait que**

le premier bouchon (9) présente une zone d'extrémité distale (29), laquelle se rétrécit dans la direction distale (X1), le premier bouchon (9) présentant à une extrémité proximale (30) une surface d'extrémité proximale (31), laquelle est conçue de manière plane, au moins une zone d'enveloppe (32) du premier bouchon (9) étant constituée d'un matériau élastique, de telle sorte que le premier bouchon (9) s'applique de manière étanche contre une paroi interne (22) du corps de seringue cylindrique creux (2).

9. Seringue (1a) selon l'une des revendications précédentes,
   **caractérisée par le fait que**
   la pièce d'extrémité cylindrique creuse (4) est réalisée sous la forme d'un cône dont le diamètre externe se rétrécit de manière continue, la pièce d'extrémité cylindrique creuse (4) étant réalisée sous la forme d'un cône Luer.

10. Seringue (1a) selon l'une des revendications précédentes,
    **caractérisée par le fait qu'**
    un moyen de perçage (33) est fixé dans le canal de passage (5) de la pièce d'extrémité (4), le moyen de perçage (33) étant relié à la pièce d'extrémité (4) au moyen d'une liaison par correspondance de matière, en particulier d'une liaison adhésive.

11. Seringue (1a) selon l'une des revendications précédentes,
    **caractérisée par le fait que**
    le corps de seringue (2) est fabriqué en verre, le corps de seringue (2) étant constitué de verre au silicate.

12. Utilisation d'une seringue (1a) selon l'une des revendications 1 à 11, pour éviter un contact du milieu (7) prévu pour l'administration avec des matériaux de contact dangereux dans la seringue pendant le stockage de la seringue, utilisation selon laquelle, dans un état de repos, la zone de réserve (6) est remplie par le milieu (7) prévu pour l'administration et dans la zone d'espacement distale (8), aucun milieu prévu pour l'administration n'est présent.

Fig. 3

Fig. 2

Fig. 1

Fig. 4

Fig. 5

Fig. 6

Fig. 7

EP 3 682 921 B1

14

Fig. 8a

Fig. 8b

Fig. 8c

Fig. 8d

EP 3 682 921 B1

Fig. 10b

Fig. 10a

Fig. 9d

Fig. 9c

Fig. 9b

Fig. 9a

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2017055462 A1
- JP 2004129695 A
- DE 10110126 A1